# EUROPEAN PATENT SPECIFICATION

(11) **EP 2 654 634 B1**
(45) Date of publication and mention of the grant of the patent: **23.03.2016**
(21) Application number: 11824272.6
(22) Date of filing: 21.12.2011
(51) Int. Cl.: A61F 5/56, A61C 7/00

(54) **DENTAL APPLIANCE FOR RESTRAINING THE TONGUE**
DENTALVORRICHTUNG ZUR BEFESTIGUNG DER ZUNGE
DISPOSITIF DENTAIRE DESTINÉ À RETENIR LA LANGUE

(30) Priority: 22.12.2010 EP 10306495; 22.12.2010 US 976489
(43) Date of publication of application: 30.10.2013
(73) Proprietor: Tongue Lab Europe, London WC2B 5DG (GB)
(72) Inventor: MAUCLAIRE, Claude, F-10000 Troyes (FR)
(74) Representative: Sutto, Luca
(86) International application number: PCT/IB2011/003268
(87) International publication number: WO 2012/085672

(56) References cited:
- WO-A1-2010/015685
- DE-C1- 19 503 288
- US-A1- 2001 027 793
- US-A1- 2009 126 742

## Description

This application is being filed on 22 December 2011, as a PCT International Patent application in the name of Tongue Laboratory Ltd., a British national corporation, applicant for the designation of all countries except the US, and Claude Mauclaire, a citizen of France, applicant for the designation of the US only.

### Cross Reference to Related Applications

The present application claims priority to U.S. Patent Application No. 12/976,489, filed December 22, 2010; and also claims priority from European Patent Application No. 10306495 filed on December 22, 2010.

### Technical Field

The present disclosure relates generally to the field of dental appliances. In particular, the present disclosure relates to a dental appliance for restraining the tongue.

### Background

Orthodontic devices such as a Quad Helix are known in the art as devices that can be attached to the molars by two bands, and have four active helix springs. These orthodontic devices can correct certain pathologies such as widening the arch of the mouth to make room for crowded teeth, or correcting a posterior cross-bite, where lower teeth are buccal (outer) than upper teeth. However, these orthodontic devices do not adequately take into account the role of the tongue in the occurrence of various pathologies.

The body of the tongue, including the mobile portion, includes a pharyngeal portion and an oral portion. The pharyngeal portion represents approximately three quarters of the length of the tongue, and the oral portion represents approximately one quarter of the length of the tongue. From front to back, the oral portion includes the tip of the tongue, an anterior central zone and a posterior zone. The posterior zone is also called the dorsum of the tongue. The anterior central and posterior zones of the tongue are laterally delimited by the lateral edges of the tongue.

The tongue is an assembly of seventeen muscles. The tongue serves to remodel all the adjoining structures of the buccal cavity, including the palate, nasal fossae, jaws, and others related structures. The pharyngeal part of the tongue begins at the hyoid bone and connects to the mandible (genioglossus muscle), the skull (styloglossus muscle), and the pharynx.

Because of untimely and unceasing action, some of these muscles develop excessively. This results in the tongue having a significant volume, greater than the volume considered to be "normal," considering the size of the buccal cavity of the patient. The condition is known as large or wide tongue.

An abnormally large tongue has at its origin dysfunctions due to poor habits often acquired in childhood at an age at which the child must stop sucking and learn to chew, speak and swallow when its first teeth emerge. One form of dysfunction consists of using the tongue to form sounds other than the articulation of dental sounds (T D N and L), another dysfunction consists of sucking or aspiring saliva, a third consists of aspirating and sucking with the tongue between teeth while swallowing an alimentary bolus instead of regular swallowing. Other types of dysfunction, such as uncontrolled and disorderly movement of the tongue for speaking, swallowing chewing and resting position of the tongue are at the origin of many osseous and dental malformations and deformations such as upper and lower prognathia and labioversion (rabbit teeth, spaces between the teeth, etc.), Down's syndrome hanging tongue, backward lower jaw, protruding upper jaw, open bite between the upper and lower jaws, narrow and deep palates with mouth breathing and even loosening of the teeth.

Reflexes, if acquired incorrectly, lead to an exaggerated use of certain muscles of the oral portion of the tongue. The tongue builds muscle in a manner that is unbalanced and excessive. The tongue progressively enlarges, working back and forth, suctioning. In such cases, the patient's palate does not widen, and remains narrow and deep, going up into the nasal fossae. Respiratory problems occur because the narrow palate reduces the width of nasal fossae and their capacity. Mouth breathing replaces normal nasal respiration, which may lead to tonsil inflammation and therefore obstruct lower respiratory tract. The back and forth motion of the tongue develops the genioglossus muscles at the inferior and anterior part of the mandible excessively, with a backwards position. The overdeveloped tongue thus serves to wrong remodeling all the adjoining structures of the buccal cavity such as the palate, nasal fossae, jaw, and other related structures, preventing or subverting development of a normal anatomy. It obstructs the respiratory tracts at the pharynx level (in particular the oropharynx).

The resting position of the tongue is also very important. A high resting position of the tongue, stuck against the palate, results in a hollowing of the palate. A hollow palate, which is large and deep, reduces the volume of the nasal fossae and blocks the entry of the oropharynx. A high resting position may press on the soft palate and soften its tensor muscle leading to snoring and reduced respiration through the nose and forced respiration through the mouth, which seems to play a role in allergic rhinitis and asthma. This is because dust (pollen, asbestos, etc.) arrives directly in the lower airways, since the air is not filtered by the nose. As with tongue dysfunctions, the tongue builds muscle in a manner that is unbalanced and excessive in this situation. Further, the tongue progressively enlarges and thickens, eventually blocking mouth breathing. Some patients may suffer both from dysfunctions and an incorrect tongue resting position.

Existing solutions for reducing snoring involve depression of the tongue to open the pharynx, thereby allowing freer breathing. One such device is discussed in European Application No. EP 2303203 A1, which generally discusses depression of the rear portion of the tongue to prevent upward or rearward movement. Such devices are generally invasive, in that they cannot easily be worn and concurrently allow normal speech and swallowing functions. Such devices also generally lack habit and particularly tongue retraining capabilities due to their intended intermittent usage.

US 2001/0027793 discloses an oral orthesis for reducing snoring and sleep apnea symptoms comprising a maxilla pallatum plate and, attached thereon, fixing means to fix the plate in the oral cavity and a tongue positioning device. Snoring results from the blocking of the airway by the tongue causing the vibrations when air is passed through. The oral orthesis includes a bar mounted in the middle of a plate and at a distance from a front part between about 0.25 and 0.8 times the length of the plate. It is pointed out that placing the bar more to the rear, i.e. at more than 0.25 times the length of the plate, would allow the tongue to be pushed in a more horizontal direction, thus more effectively opening the airway.

Still other available devices are limited regarding adjustability, and generally are configured to restrain rather than retrain the tongue functions. Such devices generally are obstructive and would prevent normal speech and/or swallowing functions, or may need to involve specific training features for unrelated issues (e.g., a Tucat's pearl).

Other existing techniques for restoring nasal breathing are highly invasive, essentially being based on surgery. But, the results obtained by these techniques are short-lived and relapses are frequent. Among the causes of these failures is that the necessary functional retraining after surgical intervention is difficult to achieve, since the patient continues to perform incorrect movements of the tongue reflexively even after surgery. It is not yet widely known and accepted that wide tongue may be a main cause for OSA or snoring.

### Summary

In accordance with the following disclosure, the above and other issues are addressed by the following:
In a first aspect, the invention relates to a dental appliance as defined in claim 1.

In a second aspect, a set of dental appliances is disclosed, including the above-referenced dental appliance. The set of dental appliances also includes a further dental appliance including an attachment mechanism to attach the appliance onto predetermined teeth of an upper jaw of a patient, and a restraining mechanism linked to the attachment mechanism. The restraining mechanism of this further dental appliance is also positioned above a patient's tongue, typically at least about 2-3 millimeters above a physiological resting position of the patient's tongue. The restraining mechanism is shaped to limit the movement of an anterior and central zone of the patient's tongue, preventing its back-and-forth motion rubbing against the hard palate. The restraining mechanism of this further dental appliance also allows the anterior tip and lateral edges of the patient's tongue to perform movements necessary for speech and swallowing.

A method of treatment of the tongue to retrain it to assume a normal resting position, leading to favorable consequences such as reduction of the volume of an enlarged tongue, is disclosed. The method includes positioning a dental appliance in a buccal cavity of a patient. The dental appliance includes an attachment mechanism to attach the appliance onto predetermined teeth of an upper jaw of a patient. The dental appliance also includes a restraining mechanism linked to the attachment mechanism, where the restraining mechanism is positioned above a patient's tongue and is shaped to limit the movement of a posterior zone of the patient's tongue, preventing the posterior zone from adhering against the patient's palate while allowing at least an anterior zone and lateral edges of the patient's tongue to perform normal physiological movements necessary for speech and swallowing. The method further includes, after a first period of time, removing the dental appliance. During the first period of time, normal tongue position is restored, leading to a reduced volume of the patient's tongue and less snoring or improved breathing or both.

A dental appliance includes an attachment mechanism to attach the appliance onto predetermined teeth of an upper jaw of a patient, and a restraining mechanism linked to the attachment mechanism. The restraining mechanism is positioned above a patient's tongue and shaped to limit the movement of an anterior central zone of the patient's tongue, preventing a back-and forth motion of the tongue rubbing against the patient's palate while allowing at least an anterior tip and posterior zone and lateral edges of the patient's tongue to perform movements necessary for speech and swallowing. The dental appliance also includes a plurality of stems extending forwardly within the patient's mouth, positioned against an edge of the palate near palatal surfaces of teeth of an upper jaw of the patient, wherein the plurality of stems apply lateral pressure against at least the predetermined teeth, thereby widening the patient's palate.

A method for treating defective high resting position usually leading to an enlarged tongue in a patient is disclosed. The method includes restraining a posterior zone of the patient's tongue to limit the movement of a posterior zone, preventing it from adhering against the patient's palate while allowing at least an anterior zone and lateral edges of the patient's tongue to perform movements necessary for speech and swallowing. The method further includes continuing the restraining for a first period of time at the end of which the volume of the patient's tongue is reduced, leading to reduced snoring and improved breathing or both, and discontinuing the restraining.

### Brief Description of the Drawings

Figure 1 is a schematic view from below of an upper jaw bearing an example of a first dental appliance of a set of dental appliances;
Figure 2 is a schematic view from above of a lower jaw illustrating the functioning position of the first dental appliance of Figure 1 relative to the patient's tongue;
Figure 3 is a sagittal section of the buccal cavity of the patient wearing the first dental appliance of Figure 1;
Figure 4 is a schematic view from below of an upper jaw bearing a second example of the first dental appliance;
Figure 5 is a perspective view, from below, of a portion of the example of Figure 4;
Figure 6 is a schematic view, from below, of the upper jaw bearing a second dental appliance of the set of dental appliances, according to a first embodiment of the present invention;
Figure 7 is a schematic view, from above, of a lower jaw illustrating the functioning position of the second dental appliance of Figure 6 relative to the patient's tongue;
Figure 8 is a sagittal section of the buccal cavity of the patient wearing the second dental appliance of Figure 6;
Figure 9 is a schematic view from below of an upper jaw bearing a second embodiment of the second dental appliance; and
Figure 10 is a perspective view, from below, of a portion of the embodiment of Figure 9.

### Detailed Description

Various embodiments of the present invention will be described in detail with reference to the drawings, wherein like reference numerals represent like parts and assemblies throughout the several views. Reference to various embodiments does not limit the scope of the invention, which is limited only by the scope of the claims attached hereto.

In general, the present disclosure relates to a set of dental appliances, including a first dental appliance and a second dental appliance. These appliances can be used individually or consecutively as a set. Generally, the first dental appliance is adapted to limit the movement of an anterior zone of the body of the tongue, while allowing the remaining portions of the tongue to perform movements necessary for speech and swallowing. However, it has little or no influence on the resting position of the posterior part of the tongue. The second dental appliance is adapted to limit a posterior zone of the body of the tongue, while allowing the rest of the tongue to perform movements necessary for speech and swallowing. Use of one or both of these dental appliances is also discussed, in particular with respect to treatment of tongue dysfunctions, tongue abnormal resting positions and of abnormally large tongue and attendant consequences, such as snoring, sleep apnea or other respiratory irregularities.

In general, in the context of the present disclosure, a normal resting position of the tongue will correspond to a position with a dorsum of the tongue in the middle of the mouth, and the tip of the tongue resting against the retroincisor palatal papilla. Notably, in a normal resting position, the space of Donders, between the hard palate and tongue, remains free.

The dental appliances of the present disclosure are described in the context of their functional position in a buccal cavity of a patient. Generally, the orientation of the figures is given by an XYZ reference frame. The x-axis corresponds to the patient's sagittal axis oriented from back to front, the y-axis corresponds to the transverse axis oriented from the patient's left to right, and the z-axis is the medial longitudinal axis, positioned substantially vertically when the patient is in the basic anatomic position, where the z-axis is oriented from bottom to top.

In some embodiments, the set of dental appliances according to this disclosure can be used in a method of treatment including the steps of placing the first dental appliance of the set on the upper jaw of a patient during a first period of time; and then, after removing the first dental appliance, placing the second dental appliance on the upper jaw of the patient during a second period of time.

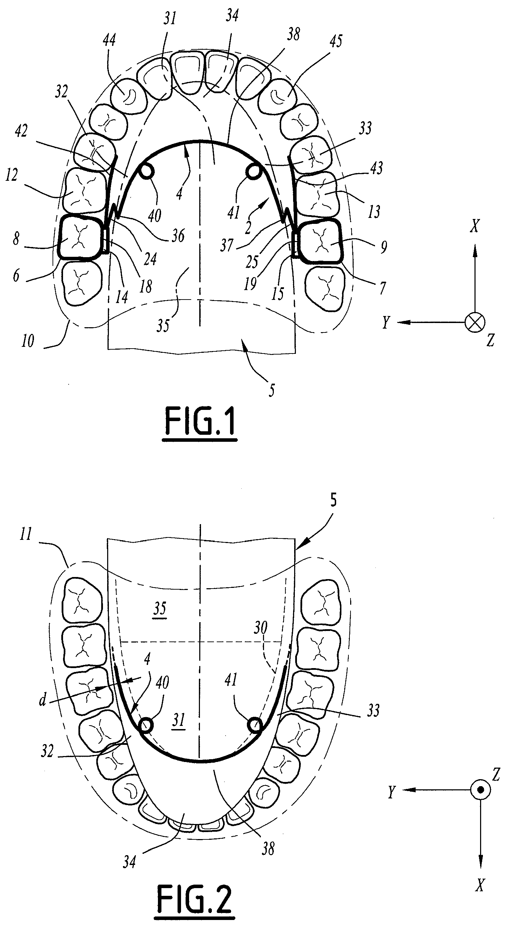

Referring now to Figures 1-3, a first dental appliance 2 is shown, within a patient's buccal cavity 1. The first dental appliance 2 is used for constraining the tongue in a set position corresponding to a normal position for swallowing and other functions of the tongue while restraining back-and-forth movement of the anterior part of the tongue which would cause it to rub against the palate, and in particular the hard palate. The first dental appliance includes a forwardly-extending arch 4 shaped and positioned for contacting a patient's tongue 5. The forwardly-extending arch 4 is shaped and positioned to limit the movements of certain portions of the tongue 5, as discussed below. In the example shown, attachment bands 6 and 7 retain the appliance 2 in position on the patient's upper jaw, and an additional means of support for stabilizing the appliance 2 during its use.

The first dental appliance 2 is specifically adapted for treatment of a patient condition relating to tongue dysfunctions, including patients having a deep, narrow palate. The first dental appliance 2 is configured, as discussed below, to enlarge the palate on one or both sides. First, it prevents the tongue from rubbing against the palate (e.g., the hard palate) and it also exerts lateral pressure on the upper jaw pushing its two sides apart to enlarge the palate.

The bands, respectively right 6 and left 7, are selected in a supplier's catalog to correspond to the size of the specific teeth selected for use, and are suited for tightening on the second molars, respectively right 8 and left 9, of the patient's upper jaw 10. As a variant, the bands 6 and 7 are arranged on the first molars 12 and 13 of the upper jaw 10. For example, this arrangement can be used in the case of children in whom the second molars are not yet emerged.

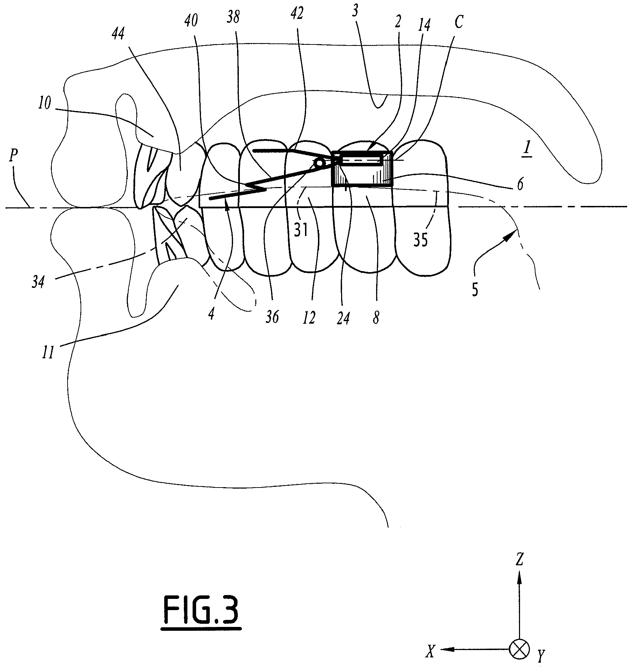

Each band 6, respectively 7, is provided with a sheath or sleeve 14, 15, whose section is substantially rectangular, which for example has dimensions of 2 mm x 2.5 mm and a length of order 4 mm. Such horizontal lingual sheaths are, for example, made of metal and are sold by the U.S.A. company Rocky Mountain Orthodontics with catalog number AO186. Each sheath 14, 15, is for example welded on a palatal surface 18, 19, of the band 6, 7, where the palatal surface is a surface oriented towards the inside of the buccal cavity 1. As shown in Figure 3, the axis C of a sheath 14, respectively sheath 15, is in a substantially horizontal plane and very close to the occlusion plane P of the patient's jaws.

The arch 4 is formed from a metal wire, for example a 0.036" diameter "Elgiloy blue" type wire, sold by the American company Rocky Mountain Orthodontics, of Denver, Colorado. Each of the ends 24 and 25 of the arch 4 is received in a corresponding sheath 14, 15, of the bands 6 and 7.

Seen from above, as illustrated in Figure 2, the arch 4 is shaped to come in contact with the border 30 of the anterior central zone 31 of the patient's tongue 5, when the patient's mouth is closed so as to limit the movements of the central zone 31 of the tongue. At the same time, the right lateral edge 32, the left lateral edge 33, the forward edge or tip 34, and the posterior zone 35 of the tongue 5 can still make the movements necessary for speech or swallowing. The arch 4 follows the arch formed by the palatal surfaces of the teeth of the lower jaw 11 at a distance d which ranges between approximately 3 cm and 0.5 cm, preferably between 3 cm and 1 cm.

In certain examples, the arch is positioned approximately 2-3 millimeters above the normal resting position of the tongue, to restrain tongue action at the anterior central portion of the tongue 5. However, variations from this guideline are possible.

In sagittal section, as shown in Figure 3, the arch 4 is located in the area of the plane of occlusion P. It should be noted that the arch 4 is away from the palate 3 of the buccal cavity 1 of the patient, thus preventing the tongue from rubbing against a portion of the palate, such as the hard palate.

The arch 4 comprises means of adjustment of the geometry thereof enabling an adaptation of the appliance 2 to the specific shape of the mouth of the patient.

The means of adjustment comprise predominantly vertical or slightly oblique loops 36 and 37, located on both sides of the sagittal plane and near the sheaths 14 and 15. In particular, the vertical loops 36, 37 can be used to adjust a height of the arch 4, thereby adjusting an amount of constraint or restraint on the tongue to accommodate patients having different physiologies. Each vertical loop 36, 37, is made by forming a loop by winding the metal wire constituting the arch 4 on itself through 360°. Although loops 36, 37 are referred to herein as vertical loops, it is recognized that these loops can, in certain examples, be partially vertical (i.e. oblique or canted with respect to a vertical plane).

When the first dental appliance 2 is in functional position in the patient's mouth, the vertical loops 36 and 37 are located in a substantially vertical plane, parallel to the XZ-plane, and directed upward. The vertical loops 36 and 37 make it possible for the dental professional to elastically deform the metal wire of the arch 4 to incline the anterior section 38 of the arch 4 more or less relative to the plane of occlusion P.

The means of adjustment also comprise two substantially horizontal right 40 and left 41 loops, placed symmetrically along the anterior section 38, on both sides of the XZ sagittal plane, about 1 cm forward from each of the vertical loops 36 and 37. The horizontal loops 40, 41 can be used to adjust the arch 4 for palate enlargement. In some examples, the horizontal loops 40, 41 are not symmetrical in position or size, In other examples, only one of such loops may be present. Although loops 40, 41 are referred to herein as horizontal loops, it is recognized that these loops can, in certain examples, be partially horizontal, i.e. oblique or canted with respect to a horizontal plane. The distance separating the two horizontal loops 40 and 41 varies depending on the width of the patient's buccal cavity and is generally from 2 to 3 cm. Each horizontal loop, 40 and 41, is made by forming a loop by winding the metal wire constituting the arch 4 on itself. The horizontal loops 40 and 41 enable the plastic deformation of the metallic wire constituting the arch 4 in order to adapt the shape of the arch 4 to the geometry of the patient's dentition and to the shape of the border 30 of the central zone of the tongue 5 with which the arch 4 comes in contact. Further, the horizontal loops 40 and 41 provide an additional contact surface between the arch 4 and the tongue 5 and make it possible to spread the palate if it is too narrow, and improve the nasal respiration.

In some examples both the vertical loops 26, 27 and the horizontal loops 40, 41 are absent; such examples would have less flexibility and less precision in arch placement as compared to the example shown.

In some other examples, either or both the vertical loops and the horizontal loops could be replaced by other adjustment means (such as micro fasteners, micro nuts and bolts, micro screws or a spring and cog mechanism etc.) and would nevertheless be included in the scope of the invention

The first dental appliance 2 preferably comprises an additional means of support. Actually, when the tongue 5 exerts forces on the arch 4, these forces, amplified because of the lever arm, are exerted on the bands 6 and 7, through the sheath 14 and 15. To compensate for these significant forces which tend to move the bands 6 and 7, the first dental appliance 2 is equipped with two stems, respectively right 42 and left 43, substantially straight. Each stem 42, 43, is constituted by a metal wire identical to that used for the arch 4. Each stem 42, 43, is attached at a first end to a band 6, 7, by insertion of this end in the sheath 14, 15 of this band. The stem 42, 43, extends from the sheath 14, 15, to which it is attached towards the front of the mouth 1, along the palatal surfaces of the upper jaw 10 pre-molars. The first end of the stem 42, 43, is located in the area of the first pre-molar. The stem 42, 43 comes to rest on a relief of the upper jaw 10 located at the limit between the enamel of the teeth and the gum.

As compared to related application PCT/EP2009/060226, the stems 42, 43 as shown extend forwardly within the patient's mouth one or two tooth lengths, as compared to the longer stems illustrated in that application. As such, the stems 42, 43 of the present application assist in widening a patient's palate, thereby further treating a sequela of a large tongue condition.

With this arrangement, when the sheaths 14 and 15 are subject to forces which tend to pivot them around an axis parallel to the y-axis, the stems 42 and 43 come to rest on the relief of the upper jaw, so as to generate forces which oppose the pivoting of the bands 6 and 8, while widening the palate.

For greater rigidity, in some examples, the first end of the stem and the end of the arch housed in the same sheath are welded together.

As a variant, a stem is made by folding the metal wire constituting the arch 4 back on itself, where the portion folded back is housed in or in any event integral with the attachment sheath.

In another variant, external parts of the stems can be bent obliquely toward the floor of the mouth. This would prevent the tongue from moving laterally and interposing between a user's teeth. However, in such examples, the distance of extension of the stems generally would not extend beyond the lower teeth.

In another variant, the arch for constraining the tongue is removable. The means for keeping the arch on the bands are consequently also adapted. For example, the palatal surface of an attachment band is provided with an element forming a sheath placed vertically, such as a Wilson 3D lingual tube with catalog number A4114 from Rocky Mountain Orthodontics of Denver, Colorado, and with which combined means provided on the corresponding end of the arch engage by insertion.

As a further variant, the vertical loops are replaced by loops arranged obliquely. Such loops enable both a height and width adjustment of the arch. It is then possible to dispense with providing the arch with horizontal loops.

To position the first dental appliance 2, the dental professional tightens the bands 6 and 7 on each of the two first molars 8 and 9 of the patient's upper jaw 10. The dental professional next lodges the ends of the arch 4 and the stems 42 and 43 in the sheaths 14 and 15 and deforms them to assure the hold by tightening. Then, using pliers, the dental professional deforms the various horizontal 40 and 41 and vertical 36 and 37 loops of the arch 4 for adapting its width and height to the geometry of the patient's buccal cavity. In its functional position, the arch 4 is adjusted a little above the desired position of the tongue, which is a normal resting position in which the tongue is relaxed and located near the dental arch of the lower jaw, just behind the lower incisors, without exerting any force on them.

At the end of the adjustment, the arch 4 is such that it leaves a sheath, downward and forward, while separating from the palatal surface of the teeth in the upper jaw so as to not interfere with the occlusion. The arch 4 is deformed so as to come into contact about 0.5 cm from the outer edge of the tongue 5. The arch 4 is therefore not arranged against the patient's palate 3, but in the space between the upper and lower arches of the patient's oral cavity.

Then the stems forming the additional supports are placed along the palatal surfaces of the premolars, near the neck of the teeth, meaning in the gum line.

In alternative arrangements, bands 6 and 7 with sheaths affixed 14 and 15 can be placed on teeth other than molars 8 and 9. In one alternative example, bands 6 and 7 with sheaths affixed 14, 15 can be placed on first molars 12 and 13. Preferably, due to pressure applied by the tongue 5 to the arch 4, the bands 6 and 7 with sheaths affixed 14, 15 are not placed on premolars.

In some examples, the sheaths 14, 15 may be fixed on other dental appliances, including those constructed from metal or resin. Example appliances can include any orthodontic or dental oral devices, such as braces or aligners.

In addition, bands 6, 7 can, in some examples, be fixed on maxillary partial or fixed dentures, for example for individuals who have lost a large number (e.g., all or nearly all) of their teeth.

Thus positioned, the appliance 2 acts by only allowing the tongue 5 the movements necessary for its normal function, meaning articulation of the alveolar sounds (T, D, N) and L, and the evacuation of the alimentary bolus and saliva by swallowing.

When the tongue 5 moves in a prohibited manner, the border 30 of the central zone meets the arch 4, which forms an obstacle. Thus, the sucking movement becomes impossible and so do other undesirable movements. To avoid injury by rubbing on the metal wire and the horizontal loop, the tongue "learns," through a reflex mechanism, to avoid certain movements and to try to remain relaxed.

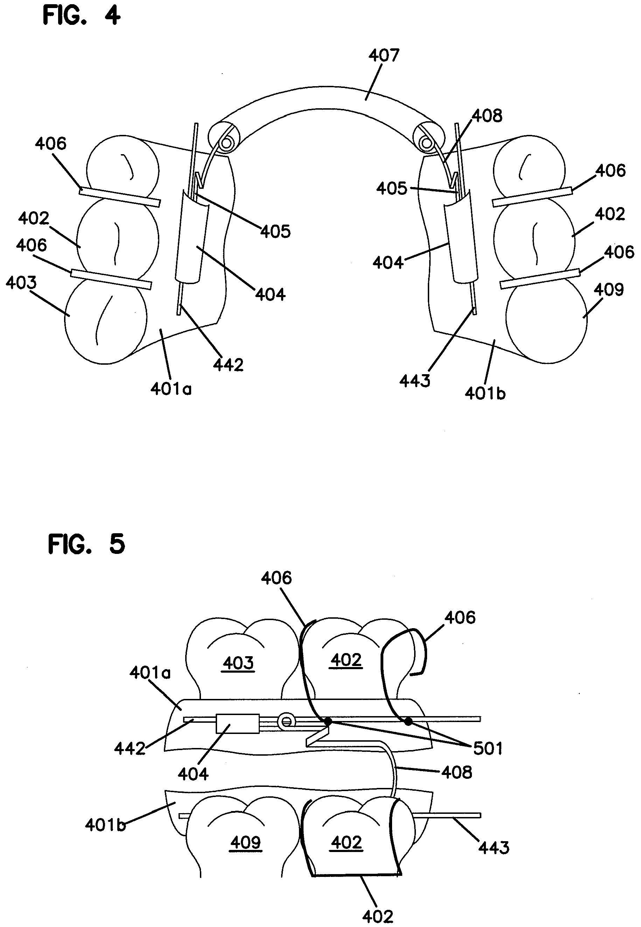

Referring to Figures 4-5, in another example of the first dental appliance, particularly suitable for patients missing one or more molars, e.g., because of a tooth extraction, or for patients willing to remove the appliance during day time for example, the means of constraint are made by a metal wire arch set in a pair of accompanying resinous formations. Referring to Figure 4, this example includes complementary left and right resinous formations 401a-b molded to fit the patient. The resinous formations 401a-b are held in place by at least two hooks 406 on each side of the upper jaw, which grip one or more available teeth, such as teeth 402, 403. Position 409 indicates a missing tooth, and Figure 4 illustrates hooks 406 grip the teeth that are adjacent to position 409. On the side of the teeth opposite from each respective resinous formation 401a-b, hooks 406 may be joined together by a cross-member, or may remain separate, for example when palate enlargement is desirable. Two sleeves 404, one on each side, are affixed by a process such as polymerization onto the resinous formations 401a-b parallel to the teeth 402, 403. The ends of the arch 405 are inserted into sleeves 404, and may be secured to sleeves 404 by a mechanical attachment such as threading, friction, or other methods known in fastening arts for this purpose. The appliance may include a sheath 407 around metal wire arch 408, the sheath 407 providing greater comfort to a user than a bare metal wire arch without a sheath.

As shown in Figure 4, stems 442, 443 are mounted within sleeves 404 on the left and right side resinous formations 401a-b, respectively. The stems 442, 443 have analogous functions to stems 42, 43 of the first dental appliance 2, cooperating with arch 408 to widen the patient's palate.

Figure 5 illustrates a detailed perspective view of a portion of the example of Figure 4, wherein hooks 406 are attached to resinous formations 401a-b at anchor points 501, one anchor point 501 per hook 406. Optional cross-member 502 connects two adjacent hooks 406 to provide additional stability.

In the example of Figures 4-5, the hooks 406 are positioned to grip teeth 402, 403, which are in one example first and second molars of the patient. In alternative examples, the hooks 406 can be positioned on resinous formations 401a-b to grip either second molars or second premolars, depending upon the geometry of the patient's mouth and the presence (or absence) or one or more teeth.

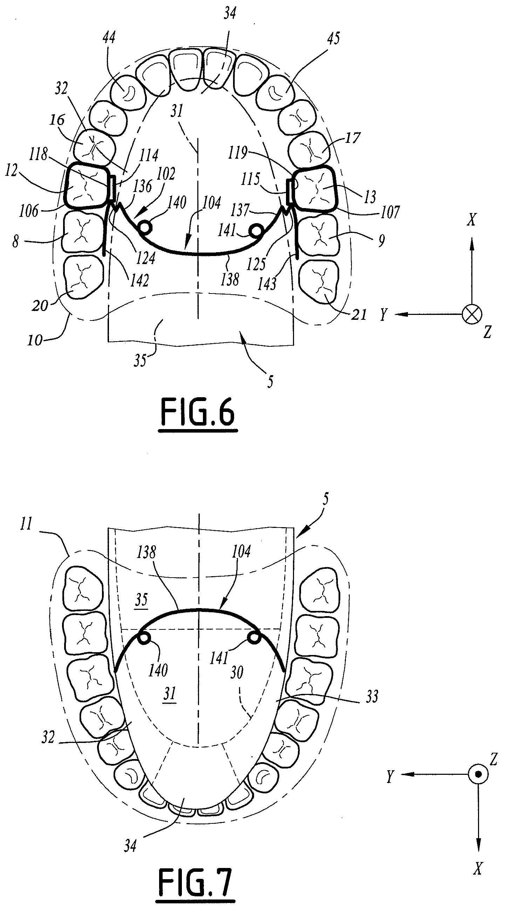

Referring now to Figures 6-8, a second dental appliance 102 of a set of dental appliances is shown, within a patient's buccal cavity 1. The second dental appliance 102 is generally used for constraining at least a posterior zone of the tongue 5 in a position corresponding to a normal resting position, i.e., one not abutting against the rear portion of the hard palate or against the soft palate, while allowing the tongue to move enough for speech and swallowing. The objective is again to retrain the tongue but this appliance seeks to correct a different defect in the tongue's positioning. Except where indicated or obvious for one skilled in the art, details of similar elements are as disclosed above with respect to the first dental appliance.

Generally, in comparing the second dental appliance 102 to the first dental appliance 2, while the first dental appliance 2 is constructed for restraining an anterior central zone 31 of the patient's tongue 5, the second dental appliance is constructed for restraining a posterior zone 35 of the tongue 5. The second dental appliance 102 is therefore particularly adapted for treatment of patients who have a resting position of his/her tongue 5 against the palate, including the rear portion of the hard palate and soft palate and uvula, but for whom palate widening is unnecessary (e.g., in instances where a correction has already been applied using the first appliance illustrated in Figures 1-5).

The second dental appliance 102 includes an arch 104 that extends rearward within the buccal cavity 1, and is suited for contacting the patient's tongue 5 for limiting the movements thereof toward the rear hard palate, the soft palate and uvula. Additional details regarding arch 104 are provided below.

In the embodiment shown, attachment bands 106, 107 are positioned on the first molars 12, 13, respectively, of the upper jaw 10. The attachment bands 106, 107 are configured for keeping the appliance 102 in position on the patient's upper jaw 10, and an additional means of support 142, 143 for stabilizing the appliance 102 during its use.

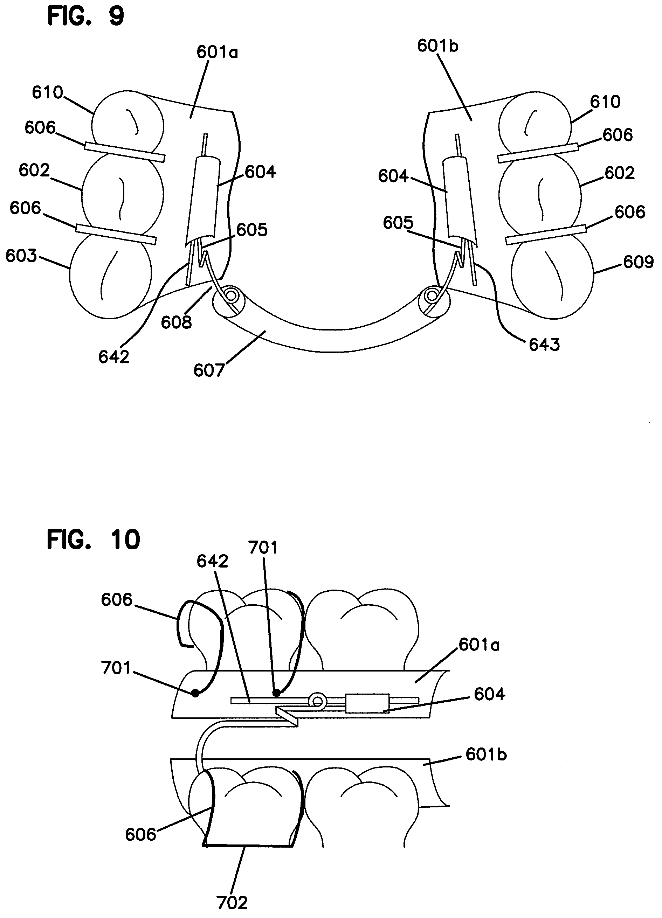

In alternative arrangements, the bands 106, 107 and associated sheaths 114 and 115 can be placed on teeth other than the first molars 12, 13. In one alternative embodiment, bands 106, 107 and associated sheaths 114, 115 can be placed on molars 8 and 9, for example where wisdom teeth 20, 21 are present. Preferably, due to pressure applied by the tongue 5 to the arch 4, the bands 106, 107 with sheaths 114, 115 are not placed on premolars. If sufficient teeth are not present, the embodiment described below in conjunction with Figures 9-10 can be used in the alternative.

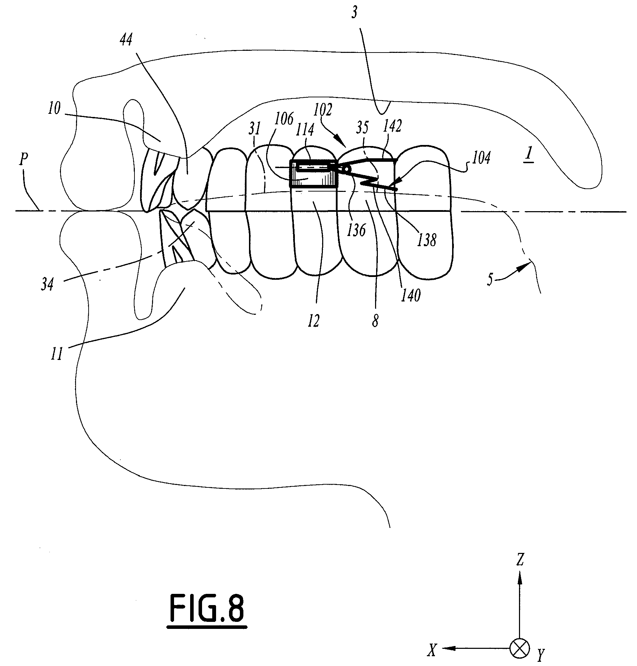

In the embodiment shown, the bands 106, 107 are suited for tightening on the first molars, respectively right 12 and left 13, of the upper jaw 10. In an alternative embodiment (not shown), the attachment bands 106 and 107 are arranged on the second pre-molars 16 and 17 of the upper jaw 10. Each band 106, 107, is provided with a sheath or sleeve 114, 115, which is for example welded on a palatal surface of the band 106, 107. Each sheath 114, 115 has a substantially rectangular cross-section, and can be of varying sizes depending upon the number and size of structures to be received into the sheath. The sheaths 114, 115 can be horizontal lingual sheaths, having dimensions of approximately 2 mm x 2.5 mm and a length of approximately 4 mm, as discussed above with respect to sheaths 14, 15 of Figures 1-3. The sheaths 114, 115 can also be welded (or otherwise affixed) on a palatal surface 118, 119 of bands 106, 107, oriented toward the inside of the buccal cavity 1. As shown in Figure 8, the axis C of a sheath 114, respectively sheath 115, is in a substantially horizontal plane and very close to the occlusion plane P of the patient's jaws.

The arch 104 is formed from a metal wire, as explained above with respect to arch 4 of Figures 1-3. Each of the ends 124 and 125 of the arch 104 is received in a corresponding sheath 114, 115, of the bands 106 and 107.

Seen from above, as illustrated in Figure 7, the arch 104 is shaped to come into contact with the posterior central zone 35 of the patient's tongue 5, when the tongue 5 tries to stick to the palate and the patient's mouth is closed so as to limit the upward movements of the posterior central zone of the tongue. At the same time, the right lateral edge 32, the left lateral edge 33, the forward edge or tip 34 and the central zone 31 of the tongue 5 can still make the movements necessary for speech or swallowing. In general, the arch 104 is positioned rearward at approximately the third molar level, such that it resides below the hard palate about 1 cm away from the front edge of the soft palate. In this way, the arch 104 prevents the tongue 5 from pressing against soft and hard palate, as well as the uvula.

In sagittal section, as shown in Figure 8, the arch 104 is located in the area of the plane of occlusion P. It should be noted that the arch 104, as with arch 4 of Figures 1-3, is away from the palate 3 of the buccal cavity 1 of the patient. When treatment is initiated, the arch 104 can be positioned relatively closer to the palate to reduce interaction and potential pain on the tongue 5. When the tongue 5 reduces in volume due to treatment, the arch 104 can be progressively lowered toward the plane of occlusion P, to further constrain the tongue in its normal working and resting positions. As such, if the arch 104 is located near the palate in an initial position, it is desirable for the arch 104 to be adjustable to gradually be spaced apart from the palate toward the plane of occlusion. For example, if a weaker or initial treatment is desired, the arch can be adjusted upward toward the palate from the top gum line plane by about 3 mm; similarly, if a stronger treatment is desired, the arch 104 can be adjusted downward up to about 3 mm below the gum line plane.

Arch 104 comprises means of adjustment of the geometry thereof, enabling an adaptation of the appliance 102 to the specific shape of the mouth of the patient. The means of adjustment comprise two substantially vertical or slightly oblique loops 136 and 137 and two substantially horizontal loops 140 and 141. As explained above, although each of these loops are referred to herein as vertical or horizontal loops, respectively, it is recognized that these loops can, in certain embodiments, be partially canted with respect to a vertical or horizontal plane, respectively.

These means enable the plastic deformation of the metallic wire constituting the arch 104 in order to adapt its shape to the geometry of the patient's dentition and to the shape of the posterior zone 35 of the tongue 5 with which the arch 104 comes into contact. Further, the horizontal loops 140 and 141 provide an additional contact surface between the arch 104 and the tongue 5.

Similarly to the first dental appliance 2, the second dental appliance 102 preferably comprises additional means of support made of two stems, respectively right 142 and left 143. The right and left stems 142 and 143, respectively, are substantially straight, and can be formed from metal wire. However, as compared to the first dental appliance 2, the stems 142, 143 of the second dental appliance 102 can in certain embodiments be further shortened, for example to a length corresponding to the width of one to one and a half teeth. The stems 142, 143 can be shortened, for example, in embodiments where the second appliance 102 is not used for enlarging the palate of a patient (e.g., the second appliance 102 is applied after treatment with the first appliance or to a patient that does not require enlarging of the palate).

The first end of each stem 142, 143, is inserted in a corresponding sheath 114, 115, alongside each end 124, 125 respectively of the arch 104. The stem 142, 143, extends from the band 106, 107, to which it is attached, towards the back of the mouth 1, along the palatal surfaces of the upper jaw molars. In the embodiment shown, the second end of the each stem 142, 143, is located in the area of the third molars 20, 21. The stems 142 and 143 come to rest on a relief of the upper jaw 10 located at the limit between the enamel of the teeth and the gum. In certain embodiments, the stems 142, 143 are approximately one tooth long, although the exact length may vary according to mouth geometry of the patient.

Additionally, each end 124 and 125 of arch 104 is received in the sheath 114, 115.

The variants encompassed for the first dental appliance 2, as discussed above, can also be applied to modify the second dental appliance 102.

In certain embodiments, the positioning of the second dental appliance 102 is done as follows.

The dental professional tightens the bands 106 and 107 on each of the two first molars 12 and 13 of the patient's upper jaw 10. The dental professional next lodges the ends of the arch 104 and the stems 142 and 143 in the sheaths 114 and 115 and deforms them to assure the hold by tightening.

Then, using pliers, the dental professional deforms the various horizontal 140 and 141 and vertical 136 and 137 loops of the arch 104 for adapting, in width and height, the geometry of the arch 104, and its posterior section 138, to the patient's oral cavity.

In its functional position, the arch 104 is such that its posterior section 138 is adjusted a little above the desired position of the tongue, which is a normal resting position in which the tongue is relaxed and located near the dental arch of the lower jaw.

At the end of the adjustment, the arch 104 is such that it leaves a sheath, downward and backward, while separating from the palatal surface of the teeth in the upper jaw so as to not interfere with the occlusion.

The arch 104 is deformed so as to come into contact with the tongue 5 when the tongue would otherwise be positioned against the palate in a resting position, but generally above a gum line plane such that it does not necessarily contact the tongue 5 when the tongue is in a normal, desired resting position. The arch 104 is therefore not arranged against the patient's palate 3, but in the space between the upper and lower dental arches of the patient's oral cavity.

Then the stems 142, 143 forming the additional supports are placed along the palatal surfaces of the molars, near the neck of the teeth, meaning in the area of the junction of the teeth with the gums.

Thus positioned, the second dental appliance 102 acts by allowing the tongue 5 only the movements necessary for its normal function, meaning articulation of the alveolar sounds T, D, N and L, and the evacuation of the alimentary bolus and saliva by swallowing.

When the tongue 5 moves in a prohibited manner or has a bad resting position fixed on the palate, the posterior zone 35 of the tongue meets the arch 104, which forms an obstacle. To avoid injury by rubbing on the metal wire and the horizontal loops, the tongue "learns," through a reflex mechanism, to avoid certain movements and to try to remain relaxed. This is the reason why the appliance 102 is referred to as providing retraining of the tongue.

Referring to Figures 9-10, an alternative embodiment of the second dental appliance is shown. This alternative embodiment is analogous to the example of Figures 4-5 relating to the first dental appliance, and is particularly adaptable for patients missing one or more molars due to tooth extraction, for patients not willing to keep a dental device inserted in their mouth throughout the day, or other reasons.

In the embodiment shown, the second dental appliance 600 includes a pair of resinous formations 601a-b. In the embodiment shown, the resinous formations 601a-b are held in place by hooks 606 on each side of the upper jaw, sized and positioned to grip one or more available teeth, in the example shown teeth 602, 603. In such embodiments, the teeth 602 are engaged by the hooks 606. In alternative embodiments, the hooks 606 and resinous formations 601a-b are adapted to grip different teeth (e.g., tooth 603), for example in the case where one or both of teeth 602 are missing. In cases where other teeth are missing, one or both of the resinous formations 601a-b can be formed around the existing teeth on the left or right sides, respectively; for example, a resin formation can take a place of a missing tooth, e.g., as part of each of the resinous formations 601a-b.

As illustrated in Figure 10, in certain embodiments, the hooks 606 are optionally joined together by a cross-member 702, which can stabilize the positions of the hooks and also assist in gripping teeth 602. However, in alternative embodiments, hooks 606 may remain separate. In the embodiment shown, the hooks 406 are attached to the resinous formations 601a-b at anchor points 501, one anchor point 501 per hook 406. Additionally, as with the device of Figures 4-5, the hooks 606 are positioned to grip teeth 602, 603, which are in one embodiment first molars of the patient. In alternative embodiments, the hooks 606 can be positioned on resinous formations 601a-b to grip either second molars or second premolars, depending upon the geometry of the patient's mouth and the presence (or absence) or one or more teeth.

In some embodiments, the sheaths 114, 115 may be fixed on other dental appliances already in place or simultaneously installed, including those constructed from metal or resin. Example appliances can include any orthodontic or dental oral devices, such as braces or aligners.

In addition, bands hooks 606 can, in some embodiments, be fixed on maxillary partial or fixed dentures, for example for individuals who have lost a large number (e.g., all or nearly all) of their teeth.

An arch 608 is generally positioned, shaped and oriented in the same manner as arch 104 of Figure 6-8. As with arch 408 of Figures 4-5, arch 608 optionally includes a sheath 607 around the metal wire of arch 608 to provide greater comfort to a user of the second dental appliance 600.

Two sleeves 604 are affixed, one on each resinous formations 601a-b, parallel to the teeth 602, 603. The sleeves 604 can be affixed to the resinous formations 601a-b by use of a polymerization process, or other equivalent methods. The ends of the arch 605 are inserted into sleeves 604, and may be secured to sleeves 604 by a mechanical attachment such as threading, friction, or other methods known in fastening arts for this purpose. The appliance may include a sheath 607 around metal wire arch 608, the sheath 607 providing greater comfort to a user than a bare metal wire arch without a sheath.

In certain embodiments, depending upon the size of the resinous formations 601a-b, sleeves 604 can be positioned on the resinous formations 601a-b, respectively, at a location adjacent to a tooth receiving hooks 606; however, in alternative embodiments, the sleeves 604 can be located further forward or backward in the mouth, for example in the case where the sleeves are adjacent to teeth 610 (or hooks are placed on teeth 610).

As shown in Figures 9-10, stems 642, 643 can extend from sleeves 604, and act to stabilize the dental appliance when pressure is applied on the arch 608 by a patient's tongue. As with stems 142, 143, stems 643, 643 are approximately one tooth long, although the exact length may vary according to mouth geometry of the patient.

Referring now to Figures 1-10 generally, during use, the first dental appliance opposes the undesirable acquired habits and hinders the dysfunction of the tongue starting from during the first days of treatment. Progressively, the tongue is re-educated so as to function properly, within the constraints of the appliance. Additionally, by stopping pressure applied by the tongue upon the palate, improved respiration through freeing the upper airways is reestablished. By forcing the tongue to move less, and in particular only in a proper manner, some muscles (e.g., the genioglossus) are less stressed and consequently, over time, their volume is reduced. By forcing the tongue to move in a proper and balanced way other muscles of the tongue regain strength and protect the capacity of the pharyngeal region. The combination of both effects enables natural breathing in the pharynx. The second appliance prevents an oversize tongue sticking to the palate and also obstructing the airways. It forces the tongue to rest in the lower part of the mouth and, over time, causes it to maintain this position which in turn frees up the airways, notably the pharynx.

In about three months of treatment with the first or second appliance, a consequent reduction of the volume of the tongue is observed. To reduce the risk of relapse, an appliance is advantageously worn for about six months. If the dental professional deems necessary to retrain the tongue's movements and position, as well as to remodel the shape of the palate, a dental appliance will be worn longer. For example, the positive action of the stems of the first dental appliance are emphasized by prolonged use, and act to further push apart the dental arches of the upper jaw, thus widening the palate.

Additionally, during treatment, the dental professional can modify the position of the arch of each of the first or second dental appliances, for example to further lower it towards the tongue as the volume of the tongue is reduced.

The use of the first or second dental appliance helps to improve articulation of words, because the presence of the appliance hinders excessive use or movement of the tongue and promotes the use of the lips. The first dental appliance widens the palate by pressing laterally on the teeth as the tongue presses on the arch. The palate is progressively remodeled and becomes less deep while also widening, which increases the volume of the nasal fossae and improves nasal respiration. Elimination of the sucking motion reduces the volume of the genioglossus muscle and reduces or eliminates double chin.

Due at least in part to the first and second dental appliances allowing normal speech and swallowing sounds, the appliances as discussed herein are suitable for continuous usage by adults, rather than being limited to night usage or to usage by children. In particular, because many adults have developed a larger tongue and deep palate, as well as a full set of developed teeth to the third molar level, the appliances discussed herein typically would be used by adults.

Since respiration is made easier, sleep apnea and snoring lessen or disappear. The patient therefore experiences deeper sleep and consequently a better quality of life, without requiring surgery. Because the first dental appliance reeducates the tongue, which models the palate, the results are more stable than these obtained with a Quad Helix.

The second appliance blocks the tongue from pressing against the palate, including the rear portion of the hard palate, the soft palate and the uvula. The tongue remains in a low and relaxed position. This also reduces the volume of the tongue and frees up the airways to make breathing easier. With the second dental appliance (e.g., dental appliance 102 or 600), the pressure exerted by the dorsum of the tongue on the appliance, when the tongue is in an untrained, high resting position, strengthens the muscles that connect the tongue to the hyoid bone and the muscles of the pharynx wall. In addition, this increases the capacity of the airways, and straightens the spine by the action on the styloglossus. The second dental appliance is therefore adapted to the treatment of a high resting position of the tongue, where the dorsum of the tongue is in contact with the palate. After a few months, the dorsum of the tongue is flattened and the oropharynx is freed.

In some applications, the dental professional uses the first and second dental appliances as a set. For example, in certain embodiments, the first and second dental appliances are used sequentially. In a first phase of the treatment, the first dental appliance is used during a first period of time, to treat the muscles of the anterior and central zones of the tongue. After the first dental appliance has been removed, in a second phase of the treatment, the second dental appliance is used during a second period, to treat the muscles of the posterior zone of the tongue. In alternative embodiments, the treatment phases of use can be reversed, with the second dental appliance used during a first period of time and the first dental appliance used during a second period of time.

The above specification, examples and data provide a complete description of the manufacture and use of the composition of the invention. Since many embodiments of the invention can be made without departing from the scope of the invention, the invention resides in the claims hereinafter appended.

## Claims

1. A dental appliance (102) comprising:
an attachment mechanism to attach the dental appliance (102) onto predetermined teeth of an upper jaw (10) of a patient; and
a constraining mechanism linked to the attachment mechanism and configured to be positioned above a patient's tongue (5), where the constraining mechanism includes an arch (104) that is shaped to limit the movement of a posterior zone (35) of the patient's tongue (5); **characterized in that**
the arch (104) is configured to be positioned above a plane of occlusion (P) of the patient, the constraining mechanism preventing the posterior zone (35) adhering to the palate (3) while allowing at least an anterior zone and lateral edges (32, 33) of the patient's tongue (5) to perform movements necessary for speech and swallowing.

2. The dental appliance (102) of claim 1, wherein the constraining mechanism is configured to be positioned at about gumline level, at least about 2 millimeters above the patient's tongue (5) when the patient's tongue (5) is in a resting position but does not touch the patient's palate (3).

3. The dental appliance (102) of claim 1 or 2, wherein the attachment mechanism comprises a first band and a second band (106, 107), the first band (106) and the second band (107) located respectively on two opposite molars (8, 9; 12, 13) or premolars (16, 17) of the upper jaw (10) of the patient, and wherein the first band (106) and the second band (107) are configured to be tightened on the two opposite molars.

4. The dental appliance (102) of claim 1 or 2 or 3, wherein the arch (104) is configured to extend rearward within a buccal cavity of the patient, the arch (104) including a metal wire, the dental appliance (102) further comprising first and second sleeves (114, 115) receiving ends (124, 125) of the arch (104).

5. The dental appliance (102) of claim 4, wherein the arch (104) includes a plurality of loops, the plurality of loops including at least one substantially horizontal loop (140, 141) and at least one substantially vertical loop (136, 137), wherein the at least one substantially horizontal loop (140, 141) enables adjustment of a width of the arch (104), and wherein the at least one substantially vertical loop (136, 137) enables adjustment of a vertical position of the arch (104).

6. The dental appliance (102) of claim 1, further comprising a plurality of stems (142, 143) extending rearward within the patient's mouth, the plurality of stems (142, 143) being configured to be positioned against an edge of the palate (3) near palatal surfaces of teeth of an upper jaw (10) of the patient.

7. The dental appliance (102) of claim 1, wherein the attachment mechanism includes a plurality of resinous formations (601 a, 601 b) having hooks (606) mounted thereto for engagement with the predetermined teeth, the dental appliance (102) further comprising a plurality of sleeves (604) mounted to the plurality of resinous formations (601 a, 601 b), the sleeves receiving ends of the constraining mechanism.

8. The dental appliance (102) of claim 7, wherein the resinous formations (601 a, 601 b) are configured for at least partially residing in a location of a missing molar of a patient.

9. The dental appliance (102) of claim 7 or 8, wherein the hooks (606) are positioned on each side of the dental appliance (102), the dental appliance (102) further comprising a cross-member (702) connecting at least two hooks (606) on each side of the dental appliance (102).

10. A set of dental appliances comprising:
a first dental appliance (2) including:
an attachment mechanism to attach the first dental appliance (2) onto predetermined teeth of an upper jaw (10) of a patient; and
a constraining mechanism linked to the attachment mechanism of the first dental appliance (2), where the constraining mechanism of the first dental appliance is shaped to limit the movement of an anterior central zone of the patient's tongue (5), preventing back-and-forth motion of the tongue (5) against the palate (3) while allowing the posterior zone (35) and lateral edges (32, 33) of the patient's tongue (5) to perform movements necessary for speech and swallowing; and
a second dental appliance which is the dental appliance (102) according to any one of claims 1 to 9.

11. The set of dental appliances according to claim 10, wherein the first dental appliance (2) further includes:
a plurality of stems (42, 43) extending forwardly within the patient's mouth, the plurality of stems (42, 43) positioned against an edge of the palate (3) near palatal surfaces of teeth of an upper jaw (10) of the patient, wherein the plurality of stems (42, 43) apply lateral pressure against at least the predetermined teeth, thereby widening the patient's palate (3).

12. The set of dental appliances according to claim 10, wherein the first dental appliance (2) restraining mechanism is configured to be positioned above a patient's tongue (5), at least about 2 millimeters above a physiological resting position of the patient's tongue (5).

13. The set of dental appliances according to claim 10, wherein the first dental appliance (2) restraining mechanism includes a forwardly-extending arch (4) shaped and positioned for contacting a patient's tongue (5).

## Patentansprüche

1. Dentalvorrichtung (102), umfassend:
einen Befestigungsmechanismus, um die Dentalvorrichtung (102) auf vorgegebenen Zähnen eines Oberkiefers (10) eines Patienten zu befestigen; und
einen mit dem Befestigungsmechanismus gekoppelten Einschränkungsmechanismus, der konfiguriert ist, über der Zunge (5) eines Patienten positioniert zu werden, wobei der Einschränkungsmechanismus einen Bogen (104) beinhaltet, der geformt ist, um die Bewegung einer hinteren Zone (35) der Zunge (5) des Patienten zu begrenzen; **dadurch gekennzeichnet, dass**
der Bogen (104) konfiguriert ist, um über einer Okklusionsebene (P) des Patienten positioniert zu werden, wobei der Einschränkungsmechanismus ein Anhaften der hintere Zone (35) am Gaumen (3) verhindert und mindestens einer vorderen Zone und seitlichen Rändern (32, 33) der Zunge (5) des Patienten erlaubt, Bewegungen zu vollführen, die zum Sprechen und Schlucken notwendig sind.

2. Dentalvorrichtung (102) nach Anspruch 1, wobei der Einschränkungsmechanismus konfiguriert ist, um ungefähr auf Höhe des Zahnfleischrands positioniert zu werden, mindestens ungefähr 2 Millimeter über der Zunge (5) des Patienten, wenn die Zunge (5) des Patienten in einer Ruheposition ist, jedoch den Gaumen (3) des Patienten nicht berührt.

3. Dentalvorrichtung (102) nach Anspruch 1 oder 2, wobei der Befestigungsmechanismus ein erstes Band und ein zweites Band (106, 107) umfasst, wobei das erste Band (106) und das zweite Band (107) jeweils auf zwei gegenüberliegenden Backenzähnen (8, 9; 12, 13) oder vorderen Backenzähnen (16, 17) des Oberkiefers (10) des Patienten liegen, und wobei das erste Band (106) und das zweite Band (107) konfiguriert sind, um auf den gegenüberliegenden Backenzähnen festgemacht zu werden.

4. Dentalvorrichtung (102) nach Anspruch 1 oder 2 oder 3, wobei der Bogen (104) konfiguriert ist, um sich rückwärtig innerhalb einer Mundhöhle des Patienten zu erstrecken, wobei der Bogen (104) einen Metalldraht beinhaltet, wobei die Vorrichtung (102) weiter erste und zweite Hülsen (114, 115) umfasst, welche Enden (124, 125) des Bogens (104) aufnehmen.

5. Dentalvorrichtung (102) nach Anspruch 4, wobei der Bogen (104) eine Mehrzahl an Windungen beinhaltet, wobei die Mehrzahl an Windungen mindestens eine im Wesentlichen horizontale Windung (140, 141) und mindestens eine im Wesentlichen vertikale Windung (136, 137) beinhaltet, wobei die mindestens eine im Wesentlichen horizontale Windung (140, 141) die Einstellung einer Breite des Bogens (104) ermöglicht, und wobei die mindestens eine vertikale Windung (136, 137) die Einstellung einer vertikalen Position des Bogens (104) ermöglicht.

6. Dentalvorrichtung (102) nach Anspruch 1, weiter umfassend eine Mehrzahl von Schäften (142, 143), die sich rückwärtig innerhalb des Mundes des Patienten erstrecken, wobei die Mehrzahl an Schäften (142, 143) konfiguriert ist, um an einem Rand des Gaumens (3) nahe zu Gaumenoberflächen von Zähnen eines Oberkiefers (10) des Patienten anliegend positioniert zu werden.

7. Dentalvorrichtung (102) nach Anspruch 1, wobei der Befestigungsmechanismus eine Mehrzahl von harzartigen Gebilden (601a, 601b) mit daran angebrachten haken (606) zum Eingriff mit den vorgegebenen Zähnen, wobei die Dentalvorrichtung (102) weiter eine Mehrzahl von Hülsen (604), angebracht an der Mehrzahl von harzartigen Gebilden (601a, 601b), umfasst, wobei die Hülsen Enden des Einschränkungsmechanismus aufnehmen.

8. Dentalvorrichtung (102) nach Anspruch 7, wobei die harzartigen Gebilde (601a, 601b) konfiguriert sind, um sich zumindest teilweise an einer Stelle eines fehlenden Backenzahns eines Patienten zu befinden.

9. Dentalvorrichtung (102) nach Anspruch 7 oder 8, wobei die Haken (606) auf jeder Seite der Dentalvorrichtung (102) positioniert sind, wobei die Dentalvorrichtung (102) weiter eine Querstrebe (702) umfasst, die mindestens zwei Haken (606) auf jeder Seite der Dentalvorrichtung (102) verbindet.

10. Satz an Dentalvorrichtungen, umfassend:
eine erste Dentalvorrichtung (2) beinhaltend:
einen Befestigungsmechanismus, um die erste Dentalvorrichtung (2) auf vorgegebene Zähne eines Oberkiefers (10) eines Patienten zu befestigen; und
einen mit dem Befestigungsmechanismus der ersten Dentalvorrichtung (2) gekoppelten Einschränkungsmechanismus, wobei der Einschränkungsmechanismus der ersten Dentalvorrichtung geformt ist, um die Bewegung einer vorderen Zentralzone der Zunge (5) des Patienten zu begrenzen, vor-und-zurück Bewegung der Zunge (5) gegen den Gaumen (3) verhindernd und der hinteren Zone (35) und seitlichen Rändern (32, 33) der Zunge (5) des Patienten, Bewegungen notwendig zum Sprechen und Schlucken zu vollführen, erlaubend; und
eine zweite Dentalvorrichtung, die die Dentalvorrichtung (102) nach einem der Ansprüche 1 bis 9 ist.

11. Satz an Dentalvorrichtungen nach Anspruch 10, wobei die erste Dentalvorrichtung (2) weiter beinhaltet:
eine Mehrzahl an Schäften (42, 43), die sich innerhalb des Mundes des Patienten nach vorne erstrecken, wobei die Mehrzahl an Schäften (42, 43) an einen Rand des Gaumens (3) nahe zu Gaumenoberflächen von Zähnen eines Oberkiefers (10) des Patienten anliegend positioniert ist, wobei die Mehrzahl an Schäften (42, 43) seitlichen Druck gegen mindestens die vorgegebenen Zähne anwenden, dadurch den Gaumen (3) des Patienten aufweitend.

12. Satz an Dentalvorrichtungen nach Anspruch 10, wobei der Beschränkungsmechanismus der ersten Dentalvorrichtung (2) konfiguriert ist, über der Zunge (5) eines Patienten positioniert zu werden, mindestens 2 Millimeter über einer physiologischen Ruheposition der Zunge (5) des Patienten.

13. Satz an Dentalvorrichtungen nach Anspruch 10, wobei der Beschränkungsmechanismus der ersten Dentalvorrichtung (2) einen sich nach vorne erstreckenden Bogen (4) beinhaltet, der geformt und positioniert ist, um die Zunge (5) eines Patienten zu kontaktieren.

## Revendications

1. Dispositif dentaire (102) comprenant :
un mécanisme d'attache pour attacher le dispositif dentaire (102) sur des dents prédéterminées d'une mâchoire supérieure (10) d'un patient ; et
un mécanisme de retenue relié au mécanisme d'attache et configuré pour être positionné au-dessus de la langue (5) d'un patient, dans lequel le mécanisme de retenue inclut une arche (104) qui est conformée pour limiter le mouvement d'une zone postérieure (35) de la langue (5) du patient ; **caractérisé en ce que**
l'arche (104) est configurée pour être positionnée au-dessus d'un plan d'occlusion (P) du patient, le mécanisme de retenue empêchant à la zone postérieure (35) d'adhérer au palais (3) tout en permettant à au moins une zone antérieure et à des bordures latérales (32, 33) de la langue (5) du patient d'exécuter des mouvements nécessaires pour parler et déglutir.

2. Dispositif dentaire (102) selon la revendication 1, dans lequel le mécanisme de retenue est configuré pour être positionné environ à un niveau de la ligne de gencive, au moins environ 2 mm au-dessus de la langue (5) du patient, quand la langue (5) du patient est dans une position de repos mais ne touche pas le palais (3) du patient.

3. Dispositif dentaire (102) selon la revendication 1 ou 2, dans lequel le mécanisme d'attache comprend une première bande et une seconde bande (106, 107), la première bande (106) et la seconde bande (107) étant placées respectivement sur deux molaires opposées (8, 9 ; 12, 13) ou deux prémolaires opposées (16, 17) de la mâchoire supérieure (10) du patient, et dans lequel la première bande (106) et la seconde bande (107) sont configurées pour être tendues sur les deux molaires opposées.

4. Dispositif dentaire (102) selon la revendication 1 ou 2 ou 3, dans lequel l'arche (104) est configurée pour s'étendre vers l'arrière à l'intérieur d'une cavité buccale du patient, l'arche (104) incluant un fil en métal, le dispositif dentaire (102) comprenant en outre un premier et un second manchon (114, 115) recevant des extrémités (124, 125) de l'arche (104).

5. Dispositif dentaire (102) selon la revendication 4, dans lequel l'arche (104) inclut une pluralité de boucles, la pluralité de boucles incluant au moins une boucle sensiblement horizontale (140, 141) et au moins une boucle sensiblement verticale (136, 137), de sorte que ladite au moins une boucle sensiblement horizontale (140, 141) permet un ajustement d'une largeur de l'arche (104), et dans lequel ladite au moins une boucle sensiblement verticale (136, 137) permet un ajustement d'une position verticale de l'arche (104).

6. Dispositif dentaire (102) selon la revendication 1, comprenant en outre une pluralité de tiges (142, 143) s'étendant vers l'arrière à l'intérieur de la bouche du patient, la pluralité de tiges (142, 143) étant configurées pour être positionnées contre un bord du palais (3) à proximité de surfaces palatales des dents d'une mâchoire supérieure (10) du patient.

7. Dispositif dentaire (102) selon la revendication 1, dans lequel le mécanisme d'attache inclut une pluralité de formations résineuses (601 a, 601b) ayant des crochets montés sur celles-ci pour engagement avec les dents prédéterminées, le dispositif dentaire (102) comprenant en outre une pluralité de manchons (604) montés sur la pluralité de formations résineuses (601a, 601b), les manchons recevant des extrémités du mécanisme de retenue.

8. Dispositif dentaire (102) selon la revendication 7, dans lequel les formations résineuses (601a, 601b) sont configurées pour résider au moins partiellement dans un emplacement d'une molaire manquante d'un patient.

9. Dispositif dentaire (102) selon la revendication 7 ou 8, dans lequel les crochets (606) sont positionnés sur chaque côté du dispositif dentaire (102), le dispositif dentaire (102) comprenant en outre un élément transversal (702) qui connecte au moins deux crochets (606) sur chaque côté du dispositif dentaire (102).

10. Groupe de dispositifs dentaires comprenant :
un premier dispositif dentaire (2) incluant :
un mécanisme d'attache pour attacher le premier dispositif dentaire (2) sur des dents prédéterminées d'une mâchoire supérieure (10) d'un patient ; et
un mécanisme de retenue relié au mécanisme d'attache du premier dispositif dentaire (2), tel que le mécanisme de retenue du premier dispositif dentaire est conformé pour limiter le mouvement d'une zone centrale antérieure de la langue (5) du patient, en empêchant un mouvement avant/arrière de la langue (5) contre le palais (3) tout en permettant à la zone postérieure (35) et aux bordures latérales (32, 33) de la langue (5) du patient d'exécuter les mouvements nécessaires pour parler et déglutir ; et
un second dispositif dentaire qui est le dispositif dentaire (102) selon l'une quelconque des revendications 1 à 9.

11. Groupe de dispositifs dentaires selon la revendication 10, dans lequel le premier dispositif dentaire (2) inclut en outre :
une pluralité de tiges (42, 43) s'étendant vers l'avant à l'intérieur de la bouche du patient, la pluralité de tiges (42, 43) étant positionnées contre une bordure du palais (3) à proximité de surfaces palatales des dents d'une mâchoire supérieure (10) du patient, dans lequel la pluralité de tiges (42, 43) appliquent une pression latérale contre au moins les dents prédéterminées, en élargissant ainsi le palais du patient (3).

12. Groupe de dispositifs dentaires selon la revendication 10, dans lequel le mécanisme de retenue du premier dispositif dentaire (2) est configuré pour être positionné au-dessus de la langue (5) d'un patient, au moins environ 2 mm au-dessus d'une position de repos physiologique de la langue (5) du patient.

13. Groupe de dispositifs dentaires selon la revendication 10, dans lequel le mécanisme de retenue du premier dispositif dentaire (2) inclut une arche (4) s'étendant vers l'avant, formée et positionnée pour venir en contact avec la langue (5) d'un patient.
